Europäisches Patentamt

European Patent Office

Office européen des brevets

(11) Numéro de publication: **0 270 413**
**A1**

## (12) DEMANDE DE BREVET EUROPEEN

(21) Numéro de dépôt: 87402482.1

(22) Date de dépôt: 04.11.87

(51) Int. Cl.⁴: **C 07 K 3/18**
C 07 K 15/06, A 61 K 35/16

(30) Priorité: 06.11.86 FR 8615471

(43) Date de publication de la demande:
08.06.88 Bulletin 88/23

(84) Etats contractants désignés:
AT BE CH DE ES FR GB GR IT LI LU NL SE

(71) Demandeur: **INSTITUT MERIEUX Société anonyme dite:**
17, rue Bourgelat
**F-69002 LYON (FR)**

(72) Inventeur: **Grandgeorge, Michel Gaston Joseph**
**12 rue du recret**
**F-69670 Vaugneray (FR)**

**Tardy, Michel**
**165 bis rue Joliot Curie**
**F-69005 lyon (FR)**

**Tayot, Jean Louis**
**1 rue des Greffières**
**F-69890 La Tour De Salvagny (FR)**

(74) Mandataire: **Bernasconi, Jean et al**
**CABINET LEMOINE ET BERNASCONI 13, Boulevard des Batignolles**
**F-75008 Paris (FR)**

(54) Procédé de préparation de facteur VIII.

(57) On met en contact le sang placentaire ou le plasma ou toute fraction plasmatique liquide contenant du facteur VIII avec une quantité de collagène I + III, on élimine la phase liquide et on met le collagène en contact, en bain ou par chromatographie, avec une solution saline pour en extraire par désorption le facteur VIII : C adsorbé.

EP 0 270 413 A1

Bundesdruckerei Berlin

## Description

Procédé de préparation de FACTEUR VIII.

La présente invention a trait à un procédé de préparation de facteur VIII à partir du plasma sanguin ou du sang placentaire ou de toute fraction ou solution contenant du facteur VIII.

On sait que le FACTEUR VIII est un complexe de deux protéines distinctes ; le VIII : Rag et le VIII : Cag. Voir HOYER L.W. : The factor VIII complex : Structure and function. Blood 58 (1), 1-12, 1981. Dans le plasma, le VIII : Rag existe sous une multitude de formes polymériques qui vont d'un poids moléculaire de 850.000 à plus de 12 millions. La protéine VIII : Cag est associée à ces divers polymères mais ne représente globalement que 1 % environ du FACTEUR VIII total. L'activité coagulante du FACTEUR VIII (VIII : C) est liée à la protéine VIII : Cag et l'activité Willebrand sur l'hémostase primaire (VIII R : R Cof) est liée aux formes polymériques de haut poids moléculaire de la protéine VIII : Rag.

Plusieurs auteurs ont étudié la propriété qu'ont divers collagènes à l'état de fibrilles d'adhérer à la protéine Willebrand du plasma sanguin. Ces travaux ont été effectués dans le cadre de l'étude du rôle joué par la protéine Willebrand dans l'adhésion des plaquettes sanguines au collagène du subendothélium des vaisseaux, phénomène précurseur qui conduit à la formation du clou hémostatique lors d'une brèche vasculaire accidentelle.

Dans NYMAN D : Interaction of collagen with the factor VIII antigen-activity - von Willebrand factor complex. Thrombosis res. 11, 433-438, 1977, a été décrite l'absorption de VIII : Rag plasmatique par un collagène commercial bovin de type I. Dans son expérience, une dose de collagène de 100 µg/ml absorbe 60 % du VIII : Rag du plasma alors que l'activité coagulante VIII : C est au contraire augmentée. LEGRAND et al. : Adsorption of factor VIII antigen-activity complex by collagen. Thrombosis res. 13, 909-911, 1978 montrent cependant qu'un collagène bovin de type I à la dose de 100 µg/ml n'adsorbe dans leur essai que 16 % du VIII : Rag et ne modifie pas le VIII : C mais qu'un collagène de type III de la même origine adsorbe 63 % du VIII : Rag et 42 % du VIII : C. Dans une publication ultérieure, NYMAN D : Von Willebrand factor dependent platelet aggregation and adsorption of factor VIII related antigen by collagen. Thrombosis res. 17, 209-214, 1980, on constate avec d'autres collagènes bovins que le collagène de type I n'adsorbe pas le VIII : Rag alors que le collagène de type III est très efficace.

D'autres auteurs s'attachent ensuite à tester diverses formes de collagène (fibres, films, aggrégats amorphes, formes réticulées chimiquement, collagènes dénaturés par la chaleur), ainsi que de divers type (I-II-III-IV et V) et diverses origines (cuir et tendons bovins, aorte porcine, peau et tendons murins, peau et placenta humains). Voir SCOTT. D.M., GRIFFIN B., PEPPER D.S. and BARNES M.J. : The binding of purified factor VIII/von Willebrand factor to collagens of different type and form. Thrombosis res. 24, 467-472, 1981 ; SANTORO S.A. : Adsorption of von Willebrand factor/factor VIII by the genetically distinct interstitial collagens. Thrombosis res. 21, 689-693, 1981 ; SANTORO S.A. et COWAN J.F. : Adsorption of von Willebrand factor by fibrillar collagen. Implications concerning the adhesion of platelet to collagen. Collagen rel. res. 2, 31-43, 1982 ; MORTON L.F., GRIFFIN B., PEPPER D.S and BARNES M.J. : The interaction between collagens and factor VIII/von Willebrand factor : Investigation of the structural requirements for interaction. Thrombosis res. 32, 545-556, 1983. Il ressort de ces études que tous les types de collagènes testés sont susceptibles d'adsorber le VIII : Rag à condition de se présenter sous une forme non dénaturée et de préférence fibrillaire. L'adsorption dépend de la surface active, d'où l'importance d'une bonne dispersion des fibrilles, au besoin à l'aide d'ondes ultrasonores.

D'après l'article précité de SANTORO et COWAN, l'adsorption des formes VIII : Rag de haut poids moléculaire mesurée par leur activité biologique cofacteur de la ristocétine sur l'aggrégation des plaquettes (VIII R : Cof) est totale à partir du plasma pour une concentration en collagène (peau humaine ou murine) d'environ 500 µg/ml ; alors que pour l'ensemble des unités VIII : Rag, l'adsorption plafonne à 82 % et que l'activité coagulante du facteur VIII (VIII : C) n'est adsorbée qu'à 55 %. Dans AIHARA M., COOPER H.A. and WAGNER R.H : Platelet-collagen interactions : Increase in rate adhesion of fixed washed platelets by factor VIII - related antigen. Blood 63 (3), 495-501, 1984, on décrit des résultats similaires avec une concentration identique d'un collagène de type I bovin (adsorption de 100 % en VIII R : Cof, 85 % en VIII : Rag et 75 % en activité VIII : C). Dans KESSLER C.M. ; FLOYD C.M., RICK M.E., KRISEK D.M., LEE S.L. and GRALNICK H.R. : Collagen - Factor VIII/von Willebrand factor protein interaction. Blood, 63 (6), 1291-1298, 1984, il est précisé qu'avec un collagène de type I bovin ou murin, on obtient seulement 68 % d'adsorption en VIII R : R Cof et environ 38 % d'adsorption en VIII : C pour une concentration en fibrilles portée à 1.000 µg/ml.

Le brevet US n° 4.331.766 décrit un procédé dans lequel du collagène de placenta est ajouté sous forme soluble à du plasma. On provoque par chauffage la formation de fibrilles qui adsorbent les FACTEURS VIII et XIII présents dans le plasma ; les fibrilles sont ensuite séparées du plasma. Le produit obtenu est utilisable comme pansement hémostatique ou comme réactif dans l'évaluation de l'aggrégabilité des plaquettes sanguines.

Les auteurs cités précédemment, s'ils ont étudié en détail les paramètres de fixation des diverses protéines et activités du FACTEUR VIII sur le collagène, ne se sont pas ou peu intéressés à la question d'une désorption ultérieure de ces facteurs. Seuls SANTORO et COWAN ainsi que KESSLER et al citent une expérimentation dans ce sens : les premiers auteurs ont pu recouvrer à l'aide de NaCl molaire 67 % de l'activité VIII R : R Cof fixée, les suivants ne retrouvent à l'aide de KBr molaire que 10 à 20 % de cette activité. Aucun de ces auteurs n'a recherché a désorber l'activité coagulante VIII : C.

Dans aucun des documents de l'art antérieur, on n'a donc pu faire état d'une séparation ultérieure sensible des différents constituants du facteur VIII qui permettrait d'obtenir un facteur VIII utilisable dans le traitement de l'hémophilie A et de la maladie de Willebrand.

La présente invention se propose de fournir un procédé de préparation de facteur VIII à partir de plasma par adsorption sur du collagène, suivi d'une désorption, qui permette de récupérer un facteur VIII purifié, et notamment un facteur VIII riche en activité coagulante VIII : C avec un grand degré de pureté, supérieur à celui des préparations classiques dites de pureté intermédiaire, la préparation obtenue par le procédé selon l'invention étant susceptible d'être utilisée pour l'administration par voie intraveineuse.

Un autre objectif de l'invention est de fournir un tel procédé qui permette de séparer l'antigène HBs Ag associé à l'hépatite B ou d'autres particules virales éventuellement présentes.

Un autre objectif de l'invention est de fournir un tel procédé qui permette d'obtenir le facteur VIII concentré et purifié soit à partir du plasma, soit à partir du sang placentaire, soit à partir de toute fraction ou solution susceptible de contenir du facteur VIII comme le cryoprécipité, la fraction I de COHN ou toute fraction intermédiaire dans les procédés de fabrication du facteur VIII

Un autre objectif est de fournir une préparation pharmaceutique contre l'hémophilie de type A.

L'invention a pour objet un procédé de préparation de facteur VIII à partir de plasma ou de sang placentaire, ou toute fraction ou solution susceptible de contenir du facteur VIII, caractérisé en ce qu'on met en contact, le plasma, le sang placentaire ou la fraction ou solution sous forme liquide (appelés ci-après "solution à traiter"), avec une quantité de collagène, de préférence humain, que l'on élimine la phase liquide puis que l'on met le collagène en contact avec une solution saline dont le pH est compris entre 5,0 et 9,0, après quoi on élimine le collagène.

L'invention a également pour objet les produits nouveaux obtenus par le procédé selon l'invention.

Le collagène utilisé peut être notamment de type I, II, III, IV, et/ou V ou leurs mélanges. On préfère le collagène I + III, notamment le collagène placentaire I + III humain qui permet d'obtenir de meilleurs résultats et qui est facile à préparer à partir du placenta humain par des techniques en soi connues combinant l'action de la pepsine et des précipitations salines.

De préférence, le contact entre la solution à traiter et le collagène s'effectue à une température qui n'est pas supérieure à 20°C.

Parmi les solutions salines envisagées, on peut citer notamment les solutions comprenant de l'acétate de sodium, du chlorhydrate de lysine et du chlorure de sodium, (NaCl) ainsi que les solutions contenant essentiellement du NaCl.

Dans un premier mode de mise on oeuvre de l'invention, le procédé peut être conduit en bain, le collagène sous forme de poudre étant repris pour une concentration comprise de préférence entre 25 ou 50 et 150 µg/unité VIII:C présente dans la solution à traiter.

De préférence, on laisse préalablement un collagène acide se dissoudre dans de l'eau désionisée avant l'adjonction à la solution à traiter.

De préférence, le plasma est dilué au 1/4 avec de l'eau désionisée pour favoriser l'adsorption du facteur VIII sur le collagène.

Le mélange est maintenu, de préférence sous agitation douce, à un pH proche de la neutralité pendant 30 à 60 mn par exemple, à une température de 10 à 15°C, par exemple. On effectue ensuite une séparation du collagène, par exemple par centrifugation, ou par filtration et on remet le collagène en suspension dans un volume suffisant d'une solution saline à pH de 5,0 à 8,0 ou 9,0. Par exemple, dans le cas du plasma, ce volume correspond au 1/5 du volume de plasma initial non dilué.

De préférence, la solution saline contient de l'acétate de sodium 0,1 M, du chlorhydrate de lysine 0,1 M, du NaCl 0,5 à 2,0 M et du citrate de sodium 0,01M.

Le surnageant obtenu est séparé, éventuellement concentré, et dialysé, stabilisé et mis sous une forme permettant son utilisation thérapeutique.

Dans un second mode de mise en oeuvre, on effectue la séparation par chromatographie sur un support chromatographique constitué d'une matrice convenable, imprégnée de collagène, la concentration de collagène étant de préférence comprise entre 2 et 10 mg/g de support. La solution à traiter est passée sur la colonne qui est ensuite rincée, après quoi on effectue une désorption du facteur retenu sur la colonne à l'aide de la solution saline. Ces opérations peuvent être effectuées à température ambiante.

Le support de chromatographique peut avantageusement être constitué d'une matrice de DEAE Dextran Spherosil (brevet FR-A n° 76.23176) modifiée et imprégnée d'un collagène placentaire humain I + III, enrichi en type III, à une teneur de l'ordre de 100 mg pour 20 g de support.

La désorption est effectuée à l'aide d'une solution saline NaCl M à 2 M.

Au cours des opérations d'adsorption et/ou de désorption précitées, on peut ajouter des agents protecteurs connus vis-à-vis de la coagulation et de la protéolyse et notamment du l'antithrombine III et/ou de l'héparine, du citrate de sodium ou de l'acide epsilon-aminocaproïque seuls ou en combinaison.

On recueille les fractions éluées qui montrent une absorption en ultra-violet. Ces fractions, éventuellement concentrées, et dialysées, sont stabilisées et mises sous une forme permettant leur utilisation thérapeutique.

On applique le produit obtenu, riche en Facteur VIII : C, à la production d'une préparation pharmaceutique anti-hémophilique A en conditionnant le produit, et de préférence en le filtrant stérilement et en le lyophilisant.

La colonne peut être régénérée par rinçage pour être réutilisée ultérieurement.

Dans chaque mode de mise en oeuvre de l'invention, on peut avantageusement soumettre le plasma initialement à une filtration pour éliminer les plaquettes et autres cellules ou débris cellulaires ou divers insolubles résiduels.

D'autres avantages et caractéristiques de l'invention apparaîtront à la lecture de la description suivante, faite à titre d'exemple non limitatif.

EXEMPLE 1

400 ml de plasma humain citraté congelé sont décongelés à 17°C et additionnés de 1150 ml d'eau désionisée à +4°C. Après ajustement du pH à 7,3 avec de l'acide chlorhydrique dilué, on ajoute 50 ml d'une solution aqueuse acide de collagène de type I + III placentaire à 800 µg/ml, ce qui correspond à un taux de collagène de 100 µg/ml de plasma. Après une agitation à l'aide d'un barreau aimanté de 30 minutes à 12°C ± 1°C, le mélange est centrifugé à 6000 tr/mn pendant 30 minutes à une température de 12°C. Le surnageant obtenu a un volume de 1580 ml ; ce surnageant est désigné par A. Le culot de centrifugation est repris par 80 ml de tampon acétate de sodium 0,1 M, chlorhydrate de lysine 0,1 M, NaCl 1 M et citrate de sodium 0,01M à pH 6,0. Après agitation de 30 minutes à température ambiante, le mélange est centrifugé à 10.000 tr/m pendant 30 minutes. Le surnageant obtenu, désigné par B, est limpide et a un volume de 78 ml pour un culot de 3 grammes.

Les résultats de dosage de protéines (mesure en absorption U.V.) et d'activité VIII : C (dosage A.P.T.T.) sont portés dans le tableau I. Le facteur VIII : C obtenu représente 50 % de la quantité initialement présente dans le plasma et est obtenu avec une activité spécifique de 1189 UI/g protéine. Cette activité spécifique correspond à celle d'un facteur VIII de haute pureté (>500 UI/g) d'après G. ROCK dans " Plasma products : Use and management - A Technical Workshop" édité par J. KOLLINS, A.F.H. BRITTEN et A.J. SILVERGLEID - 1982.

## TABLEAU I

| | Volume mL | Protéines | | ACTIVITE VIII : C | | | Activité spécifique UI/g |
|---|---|---|---|---|---|---|---|
| | | g1/ | g totaux | UI/ml | UI/totales | % plasma | |
| PLASMA DEPART | 400 | ND | ND | 0,910 | 364 | 100 | ND |
| SURNAGEANT A (FACTEUR VIII non adsorbé) | 1580 | ND | ND | 0,025 | 39 | 11 | ND |
| SURNAGEANT B FACTEUR VIII adsorbé et élué | 78 | 1,96 | 153 | 2,33 | 182 | 50 | 1189 |

EXEMPLE 2

250 ml de plasma citraté congelé sont décongelés à 13°C et additionnés de 250 ml de tampon citrate de sodium 10 mM, epsilon-aminocaproate de sodium 20 g/l-pH 7,0 contenant 0,4 UI/ml d'héparine. On injecte cette solution sur une colonne contenant 20 g du support de collagène constitué d'une matrice de DEA Dextran Sphérosil (billes de silice) modifié, imprégnée de 100 mg du collagène placentaire de l'exemple 1, ce qui correspond à une quantité de collagène de 400 µg/ml de plasma. La colonne a été équilibrée auparavant dans un tampon citrate 0,01 M, NaCl 3 g/l, epsilon-aminocaproate de sodium 10 g/l, pH 7,0. Après injection du plasma dilué, on rince la colonne à l'aide du tampon d'équilibrage contenant en plus 0,4 UI/ml d'héparine.
- On recueille au total un volume de plasma dilué adsorbé de 520 ml.
-L'élution du FACTEUR VIII est obtenue à l'aide d'un tampon citrate 0,01 M, NaCl 1,5 M, epsilon-aminocaproate de sodium 10 g/l pH 7,0 contenant 0,4 U/ml d'héparine. Les fractions correspondant à la sortie du pic de protéines sont recueillies et représentent un total de 76 ml.
-Le débit d'injection des différentes solutions sur la colonne dans cet exemple est de 180 ml/h pour une colonne de diamètre 2,5 cm et hauteur 9,5 cm.

- Les différentes fractions : plasma dilué de départ, plasma dilué adsorbé, facteur VIII élué sont analysées vis-à-vis du taux de protéines (absorption U.V.) et du taux de facteur VIII : C (dosage A.P.T.T. ). Les résultats sont portés dans le tableau II. Le facteur VIII : C obtenu représente 32 % de la quantité initialement présente dans le plasma et est obtenu avec une activité spécifique de 2.220 UI/g protéine.

## TABLEAU II

| | Volume ml | Protéines g/ | g totaux | ACTIVITE VIII : C UI/ml | UI totales | % plasma | Activité spécifique UI/g |
|---|---|---|---|---|---|---|---|
| PLASMA DILUE DEPART | 500 | ND | ND | 0,57 | 285 | 100 | ND |
| PLASMA DILUE ADSORBE | 520 | ND | ND | 0,055 | 28 | 9,8 | ND |
| FACTEUR VIII ELUE | 76 | 0,54 | 41 | 1,20 | 91 | 32 | 2.220 |

EXEMPLE 3

Cette expérience est effectuée pour illustrer la séparation de l'antigène HBsAg du FACTEUR VIII dans le protocole de purification en colonne décrit dans l'exemple 2. Les paramètres de travail indiqués dans l'exemple 2 ont été repris exactement. On utilise comme plasma de départ un mélange de plasma congelé/décongelé standard (200 ml) et de plasma congelé/décongelé provenant d'un donneur porteur de l'antigène HBsAg (50 ml). A la fin de l'expérience, les différentes fractions, plasma dilué de départ, plasma dilué adsorbé, FACTEUR VIII élué sont analysées vis-à-vis de l'antigène HBsAg (dosage radioimmunologique ABBOTT). Les résultats sont portés dans le tableau III. Ils montrent que l'éluat de FACTEUR VIII ne contient plus que 0,1 % des molécules HBsAg présentes dans le plasma, soit une purification de 3 log 10.

## TABLEAU III

| | Volume ml | Concentration HBsAg ng/ml | Total HBsAg ng | % plasma |
|---|---|---|---|---|
| PLASMA DILUE DEPART | 496 | 140 | 69.440 | 100 |
| PLASMA DILUE ADSORBE | 630 | 120 | 75.600 | 100 |
| FACTEUR VIII ELUE | 170 | 0,4 | 68 | 0,1 |

* limite de détection du test : 0,3 ng/ml

## EXEMPLE 4

Du cryoprécipité, obtenu par congélation et décongélation de plasma sanguin humain, est extrait à température ambiante par de l'eau distillée. L'extrait, riche en facteur VIII, est purifié de manière classique par adsorption sur du gel d'alumine Après centrifugation, on obtient 600 ml de surnageant qui contient 14,3 UI/ml de facteur VIII : C avec une activité spécifique de 1.140 UI/g de protéine. Le pH de cette solution est ajusté à 6,8 et on effectue une filtration sur membrane 0,45 micron. On ajuste ensuite le pH à 7,3 et la température à 10°C puis on ajoute 600 ml d'une solution aqueuse acide de collagène de type I + III placentaire à 1 mg/ml. Après une agitation douce de 30 minutes à 10°C le mélange est centrifugé. Un précipité de 30 g est obtenu qui est dispersé dans une solution à pH 6 de NaCl M, citraté de sodium 20 mM et glycocolle 100 mM. Après un contact de 1 h à température de 25°C, le mélange est centrifugé. Le surnageant est récupéré, ajusté à pH 6,8 et filtré sur membrane 0,2 micron puis concentré par ultrafiltration et diafiltré par 2,5 volumes de tampon citrate 20 mM, glycocolle 100 mM, lysine 150 mM, pH 6,8, sur une membrane de 10 000 Dalton. On obtient une solution qui contient 36 UI/ml d'un facteur VIII : C de très haute pureté qui possède une activité spécifique de 9.550 UI/g protéine. La préparation obtenue est stable à une température ambiante pendant au moins 24 heures. Elle peut être filtrée stérilement et lyophilisée pour être utilisée comme médicament de substitution chez les hémophiles A déficients en facteur VIII : C.

## Revendications

1. Procédé de préparation de facteur VIII à partir de plasma ou de sang placentaire, ou toute fraction liquide ou solution susceptible de contenir du facteur VIII, caractérisé en ce que l'on met en contact le plasma ou le sang placentaire ou la fraction avec une quantité de collagène, notamment humain, que l'on élimine la phase liquide, puis que l'on met le collagène en contact avec une solution saline dont le pH est compris entre 5,0 et 9,0, après quoi on élimine le collagène.

2. Procédé selon la revendication 1 caractérisé en ce que l'on utilise du collagène placentaire I + III, notamment enrichi en type III.

3. Procédé selon l'une des revendications 1 et 2, caractérisé en ce que le plasma est dilué au 1/4 avec de l'eau désionisée avant mise en contact avec le collagène.

4. Procédé selon l'une quelconque des revendications 1 à 3, caractérisé en ce que le collagène est utilisé sous forme d'une solution aqueuse acide.

5. Procédé selon l'une quelconque des revendications 1 à 4, caractérisé en ce que la solution saline a un pH de 6,0.

6. Procédé selon l'une quelconque des revendications 1 à 5, caractérisé en ce que la solution saline contient du NaCl, notamment à une concentration de 0,5 M à 2 M.

7. Procédé selon la revendication 6, caractérisé en ce que la solution saline contient également de l'acétate de sodium, du chlorhydrate de lysine et du citrate de sodium.

8. Procédé selon la revendication 7, caractérisé en ce que la solution saline contient: NaCl 0,5 M à 2 M, acétate de sodium 0,1 M et chlorhydrate de lysine 0,1 M et citrate de sodium 0,01 M.

9. Procédé selon l'une quelconque des revendications 1 à 8, caractérisé en ce que les étapes du procédé sont conduites en bain.

10. Procédé selon la revendication 9, caractérisé en ce que la quantité de collagène est amenée à une concentration comprise entre 25 ou 50 μg et 150 μg par unité coagulante VIII:C présente dans la solution à traiter.

11. Procédé selon l'une des revendications 9 et 10, caractérisé en ce que l'on maintient la phase liquide en contact avec le collagène sous agitation douce à pH proche de la neutralité, que l'on élimine la phase liquide et que l'on remet le collagène en suspension dans une solution saline à pH de 5,0 à 9,0, après quoi on recueille le surnageant.

12. Procédé selon l'une quelconque des revendications 1 à 8, caractérisé en ce que l'on effectue une chromatographie du plasma ou du sang placentaire ou de toute fraction liquide ou solution susceptible de contenir du facteur VIII sur un support imprégné de collagène, après quoi on procède à une élution à l'aide de la solution saline.

13. Procédé selon la revendication 12, caractérisé en ce que le support de chromatographie est constitué d'une matrice de DEAE Dextran Sphérosil modifié imprégnée de collagène à une teneur de l'ordre de 100 mg/20 g de support.

14. Procédé selon l'une des revendications 12 et 13, caractérisé en ce que l'on effectue l'élution à l'aide d'une solution de NaCl M à 2 M.

15. Procédé selon l'une quelconque des revendications 1 à 14, caractérisé en ce que l'on ajoute des agents protecteurs du facteur VIII vis-à-vis de la coagulation et de la protéolyse, notamment de l'antithrombine III, de l'héparine de l'acide epsilon-aminocaproïque ou du citrate de sodium.

16. Produit riche en facteur VIII : C purifié, caractérisé en ce qu'il est obtenu par le procédé selon l'une quelconque des revendications 1 à 15.

17. Application du produit selon la revendication 16 à la production d'une préparation pharmaceutique

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X,D | COLLAGEN REL. RES., vol. 2, 1982, pages 31-43; S.A. SANTORO et al.: "Adsorption of von Willebrand factor by fibrillar collagen - implications concerning the adhesion of platelets to collagen" * Page 31, ligne 11 - page 32, ligne 4; page 32, lignes 30-33; page 34, lignes 2-11; page 35, ligne 15 - page 36, ligne 7; page 37, ligne 17 - page 38, ligne 3 * --- | 1,6,7 | C 07 K    3/18 C 07 K   15/06 A 61 K   35/16 |
| A,D | BLOOD, vol. 63, no. 6, juin 1984, pages 1291-1298, Grune & Stratton, Inc.; C.M. KESSLER et al.: "Collagen-factor VIII/von Willebrand factor protein interaction" * Page 1291, colonne de droite, alinéa 1; page 1292, colonne de gauche, alinéa 3; page 1293, colonne de gauche, alinéa 3; page 1296, colonne de droite, alinéa 2 * --- | 1-11 | |
| A | CHEMICAL ABSTRACTS, vol. 101, no. 19, 5 novembre 1984, page 448, résumé no. 168119y, Columbus, Ohio, US; M. AIHARA et al.: "Interaction of factor VIII complex with collagen", & KETSUEKI TO MYAKKAN 1984, 15(3), 234-40 * Résumé * --- | 1,12 | **DOMAINES TECHNIQUES RECHERCHES (Int. Cl.4)** A 61 K |
| A,D | EP-A-0 023 607  (BEHRINGWERKE AG) * Page 1, lignes 1-5,22-24; page 3, lignes 4-10,15-22; revendications 1,3 * ---                           -/- | 1 | |

Le présent rapport a été établi pour toutes les revendications

| Lieu de la recherche | Date d'achèvement de la recherche | Examinateur |
|---|---|---|
| LA HAYE | 11-02-1988 | CHARLES D.J.P.I.G. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

& : membre de la même famille, document correspondant

Office européen des brevets

**RAPPORT DE RECHERCHE EUROPEENNE**

Numero de la demande

EP 87 40 2482

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | THROMBOSIS RESEARCH, vol. 43, no. 2, 1986, pages 213-218, Pergamon Journals Ltd.; T. KIKUCHI et al.: "Interaction between plasma factor VIII and collagen" ----- | | |

DOMAINES TECHNIQUES RECHERCHES (Int. Cl.4)

Le présent rapport a été établi pour toutes les revendications

| Lieu de la recherche | Date d'achèvement de la recherche | Examinateur |
|---|---|---|
| LA HAYE | 11-02-1988 | CHARLES D.J.P.I.G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant